# EUROPÄISCHE PATENTSCHRIFT

(11) **EP 2 624 752 B1**
(45) Veröffentlichungstag und Bekanntmachung des Hinweises auf die Patenterteilung: **03.12.2014**
(21) Anmeldenummer: 11813667.0
(22) Anmeldetag: 22.09.2011
(51) Int. Cl.: A61B 5/16, A61B 5/11

(54) **VERFAHREN UND VORRICHTUNG ZUR ERFASSUNG DER GEMÜTSZUSTÄNDE EINES PFERDES**
METHOD AND APPARATUS FOR SENSING A HORSE'S MOODS
PROCÉDÉ ET DISPOSITIF POUR DÉTERMINER L'HUMEUR D'UN CHEVAL

(30) Priorität: 06.10.2010 DE 102010038028
(43) Veröffentlichungstag der Anmeldung: 14.08.2013
(73) Patentinhaber: Xybermind GmbH, 72072 Tübingen (DE)
(72) Erfinder: LÖSCHINGER, Jürgen, 72072 Tübingen (DE)
(74) Vertreter: Habbel, Ludwig
(86) Internationale Anmeldenummer: PCT/DE2011/075230
(87) Internationale Veröffentlichungsnummer: WO 2012/048704

(56) Entgegenhaltungen:
- WO-A1-2009/058004
- DE-A1- 19 834 257
- GB-A- 2 437 250
- CAROLE FUREIX ET AL: "What do ears positions tell us about horse welfare?", PROCEEDINGS OF THE 45TH CONGRESS OF THE INTERNATIONAL SOCIETY FOR APPLIED ETHOLOGY (ISAE): SCIENTIFIC EVALUATION OF BEHAVIOR, WELFARE AND ENRICHMENT, 31. JULI - 04. AUGUST 2011, INDIANAPOLIS, USA, 2011, Seite 35, XP55025463, DOI: 10.3921/978-90-8686-737-0
- A. BERGER ET AL: "Evaluation of living conditions of free-ranging animals by automated chronobiological analysis of behavior", BEHAVIOR RESEARCH METHODS, INSTRUMENTS, & COMPUTERS, Bd. 35, Nr. 3, 1. August 2003 (2003-08-01) , Seiten 458-466, XP55025429, ISSN: 0743-3808
- S.J. HOBBS ET AL: "Motion analysis and its use in equine practice and research", WIENER TIERÄRZTLICHE MONATSZEITSCHRIFT, Bd. 97, 1. Januar 2010 (2010-01-01), Seiten 55-64, XP55025423,

## Beschreibung

Die Erfindung betrifft ein Verfahren zur Erfassung der Gemütszustände eines Pferdes.

Aus der US 5,138,550 A ist es bekannt, den Gang eines Pferdes anhand am Pferdekörper angebrachter Bewegungssensoren zu erfassen, so dass Gangfehler ermittelt werden können.

Auch aus der US 7,467,603 B2 ist es bekannt, bewegungsabhängige Parameter durch am Pferdekörper angebrachte Senso-ren zu erfassen, so dass aufgrund der daraus gewonnenen Erkenntnisse zur Beweglichkeit des Pferdes das Pferd selbst und / oder sein Reiter vor Schäden geschützt werden kann oder anhand der gewonnenen Daten das Pferd identifiziert werden kann.

Aus der WO 2006/053290 A2 ist es bekannt, den Zusammenhang von Atmung und Bewegung eines Pferdes anhand am Pferdekörper angebrachter Bewegungssensoren zu erfassen, wobei ein Beschleunigungssensor die durch die Atmung hervorgerufenen Bewegungen im Atemsystem des Pferdes erfasst.

Aus der WO 2004/084624 A1 ist es bekannt, die Fitness eines Pferdes durch den Zusammenhang von Kreislaufdaten eines Pferdes und Ortsdaten zu beurteilen, indem beispielsweise GPS-gestützt die Geschwindigkeit des Pferdes ermittelt wird und mit zeitgleich erfassten physiologischen Daten des Pferdes verglichen wird, beispielsweise der Herzfrequenz, dem Blutdruck, der Körpertemperatur oder dergleichen.

Auch die US 6,952,912 B2 schlägt vor, die Atmung eines Pferdes zu analysieren, indem Atmungsgeräusche erfasst werden. So sollen Anomalien der Atemwege oder auch das athletische Potential des Pferdes erkannt werden. Die Ausgestaltung des Sensors soll die Ausschaltung von Störeinflüssen durch Umweltgeräusche wie Wind, die Hufe oder dergleichen ermöglichen.

Mit einer etwas anderen Zielsetzung als die vorgenannten Schriften, bei denen es um die Bewertung des körperlichen Zustandes des Pferdes geht, schlägt die DE 198 34 257 C2 ein Verfahren zum Überwachen eines Tieres und insbesondere eines Pferdes vor, bei dem ebenfalls unterschiedliche Parameter anhand am Pferdekörper angebrachter Sensoren erfasst werden. In den dazu verwendeten Messeinrichtungen sind Soll-Werte der entsprechenden Parameter abgespeichert, die mit den aktuell erfassten Messwerten verglichen werden. Im Unterschied zu den vorgenannten Schriften ist es gemäß der DE 198 34 257 C2 jedoch auch möglich, außer rein physiologischen Aussagen, wie zu den Bewegungen oder der Schweißausbildung des Pferdes, auch Aussagen über das Wohlbefinden des Pferdes zu erhalten, so dass diese Druckschrift ein gattungsbildendes Verfahren beschreibt.

Der Erfindung liegt die Aufgabe zugrunde, ein gattungsgemäßes Verfahren dahingehend zu verbessern, dass durch die Überwachung des Gemütszustandes eines Pferdes eine Aussage über dessen Lebens- bzw. Haltungsbedingungen getroffen werden kann, um ggf. diese Bedingungen verbessern zu können.

Diese Aufgabe wird durch ein Verfahren nach Anspruch 1 gelöst.

Die Erfindung ist in den Ansprüchen definiert.

Die Erfindung schlägt mit anderen Worten vor, eine differenzierte Aussage über wenigstens zwei Gemütszustände des Pferdes zu ermöglichen, so dass nicht nur das Verhalten des Pferdes im Training oder unter einer abgerufenen Leistung beurteilt werden kann, sondern generell die Lebensumstände des Pferdes beurteilt werden können. Während die DE 198 34 257 C2 eine quantitative Differenzierung eines einzigen qualitativen Kriteriums - also letztlich nur eines einzigen Gemütszustandes - vorschlägt, nämlich betreffend das Wohlbefinden die beiden Aussagen "das Pferd fühlt sich wohl" oder "das Pferd fühlt sich nicht wohl" ermöglicht, werden vorschlagsgemäß zu wenigstens zwei qualitativ unterschiedlichen Gemütszuständen des Pferdes Aussagen gegeben. Diese unterschiedlichen Gemütszustände können beispielsweise Angst, Müdigkeit, Aggression, Anspannung bzw. Entspannung, Interesse bzw. Desinteresse oder Wut sein, zu denen jeweils eine quantitative Aussage ermöglicht wird.

Die Umsetzung von Sensorinformationen in Gemütszustände erfolgt in mehreren Schritten. In einem ersten Schritt müssen die verschiedenen körpersprachlichen Signale anhand der Sensordaten erkannt werden. Je nachdem, ob es sich dabei um ein statisches körpersprachliches Signal (z. B. Ohrstellung) oder um ein dynamisches körpersprachliches Signal (z. B. Zucken der Ohren) handelt, kommen dafür unterschiedliche Verfahren zum Einsatz. Danach wird jedes erkannte körpersprachliche Signal je nach seiner Ausprägung quantifiziert. Diese Werte werden in einer weiteren Bearbeitungsstufe in detaillierte Gefühle und Stimmungen umgerechnet. Aus diesen einzelnen Gemütslagen werden dann umfassendere Aussagen, wie z. B. das Wohlbefinden berechnet, wie später noch detaillierter ausgeführt wird.

Die Erfindung geht davon aus, dass bereits die Haltung der Ohren und / oder des Schweifs die Gemütslage eines Pferdes - also die Gemengelage aus den mehreren einzelnen Gemütszuständen z. B. wie oben erwähnt Angst, Müdigkeit und dergleichen - gut ausdrücken kann:
Befinden sich die Ohren in einer Grundstellung, also aufgerichtet und in einer seitlich leicht nach vorne weisenden Ausrichtung, so kann davon ausgegangen werden, dass das Pferd sich in einem entspannten Zustand befindet.

Nimmt das Pferd ein Geräusch wahr, so wird es eines oder beide Ohren in Richtung des Geräusches drehen, so dass diese Ohrenstellung bzw. die Ohrenbewegung anzeigen können, dass das Pferd einen Gemütszustand hat, der mit "aufmerksam" bewertet werden kann.

Hört das Pferd ein beunruhigendes Geräusch, so werden die Ohren gespitzt und ggf. zusätzlich der Kopf oder der gesamte Körper des Pferdes in die betreffende Richtung des Geräusches gedreht. Durch die Anordnung zusätzlicher Sensoren kann diese Körper- oder Kopfbewegung erfasst werden und ggf. auch die Ausrichtung des Pferdes in einem Raum erfasst werden, so dass eine Aussage über die entsprechende Unruhe, durch welche die Gemütslage des Pferdes gekennzeichnet ist, ausgegeben werden kann.

Hängen die Ohren entspannt seitwärts nach unten, so spricht dies dafür, dass das Pferd döst und entspannt oder teilnahmslos ist.

Hängen die Ohren schlaff nach unten, so ist es wahrscheinlich, dass das Pferd Schmerzen empfindet.

Wenn die Ohren gespitzt und steil aufgerichtet sind und zudem zucken oder flackern, so empfindet das Pferd Panik.

Wenn die Ohren rückwärts flach an den Kopf angelegt sind, so strahlt das Pferd Aggressivität oder Dominanz aus.

Bereits allein durch die sensorische Erfassung der Ohrenstellung kann daher eine vergleichsweise differenzierte Aussage anhand der über die Ohren vermittelten körpersprachlichen Signale des Pferdes über den Gemütszustand des Pferdes ausgegeben werden.

Im einfachsten Fall kann ein Sensor an einem Ohr verwendet werden, wenn davon ausgegangen werden kann, dass beide Ohren stets synchron bewegt werden. Vorzugsweise jedoch werden die Bewegungen beider Ohren erfasst, indem an jedem Ohr ein eigener Sensor vorgesehen ist.

Der Sensor kann als entfernbarer Ohrclip, oder als fest angebrachte Ohrmarke ausgestaltet sein. Insbesondere vorteilhaft kann er in einen Ohrschützer integriert sein, wie er aus dem Pferdesport ohnehin bekannt und handelsüblich ist.

Um die Haltung der Ohren korrekt zu erfassen und nicht durch überlagerte Kopfbewegungen zu Fehlern in der Auswertung der Ohrbewegungen zu kommen, kann vorzugsweise ein weiterer Sensor in der Nachbarschaft des Ohrs vorgesehen sein, vorzugsweise am Kopf des Pferdes, so das durch einen Vergleich beider Bewegungen die Haltung des Ohrs am Kopf präzise berechnet werden kann.

Wenn der Ohrensensor ohnehin in einem Ohrenschützer angeordnet ist, kann dieser Ohrenschützer so ausgestaltet sein, dass er auch einen dem Ohr benachbarten Teil des Pferdekopfes bedeckt, so dass der oben erwähnte zweite, dem Ohr benachbarte Sensor in diesem Teil des Ohrenschützers angeordnet sein kann, der sich nicht zusammen mit dem Ohr bewegt, sondern ortsfest seine Lage am Kopf beibehält.

In an sich bekannter Weise stehen für die Speicherung und die Auswertung der Messdaten mehrere bekannte Wege zur Verfügung, und auch die Anzeige der Aussage über den Gemütszustand des Pferdes kann in an sich bekannter Weise auf unterschiedliche Art ermöglicht werden.

Die Verarbeitung der von einem oder mehreren Sensoren erfassten Messdaten kann beispielsweise unmittelbar in einem Gerät erfolgen, das am Pferd angeordnet ist, oder sie kann auf einem zentralen Server erfolgen, zu dem sie übertragen werden. Diese Übertragung kann sofort erfolgen, insbesondere drahtlos, so dass nahezu in Echtzeit die Aussage über die momentane Befindlichkeit des Pferdes möglich ist. Die Übertragung der Messdaten kann jedoch alternativ auch derart erfolgen, dass diese Daten zunächst in einem am Pferd angeordneten Gerät zwischengespeichert und später ausgelesen werden, wobei das Auslesen ebenfalls drahtlos oder dadurch erfolgen kann, dass das erwähnte Gerät mittels einer elektrischen Kontaktierung-wie beispielsweise eine USB Kabelverbindung - an ein Auslesegerät - wie beispielsweise einen PC - angeschlossen wird.

Die Auswertung der Messdaten kann in dem Auslesegerät selbst erfolgen, z. B. wenn dies ein PC ist, auf dem ein entsprechendes Auswertungsprogramm läuft, oder die Messdaten können vom Auslesegerät zu einem Rechner weitergeleitet werden, auf dem als Expertensystem ein Softwareprogramm zur Messdatenauswertung läuft, wobei dieses Expertensystem über beispielsweise das Internet räumlich weit entfernt von dem betreffenden Pferd angeordnet sein kann, z. B. in einer anderen Stadt, einem anderen Staat oder einem anderen Kontinent.

Die Ausgabe der getroffenen Aussage über den Gemütszustand des Pferdes kann lokal erfolgen, beispielsweise dort, wo der Server steht, auf dem die Messdaten ausgewertet werden, also beispielsweise in einem Reitstall. Die Ausgabe der Aussage über den Gemütszustand kann jedoch alternativ dazu oder ergänzend weitergeleitet werden, beispielsweise als eMail oder als SMS zu dem Pferdebesitzer, Pferde-Trainer, Tierarzt, Reitstallbesitzer oder dergleichen, wo diese Aussage z. B. auf einem PC oder auf einem mobilen Gerät angezeigt werden kann.

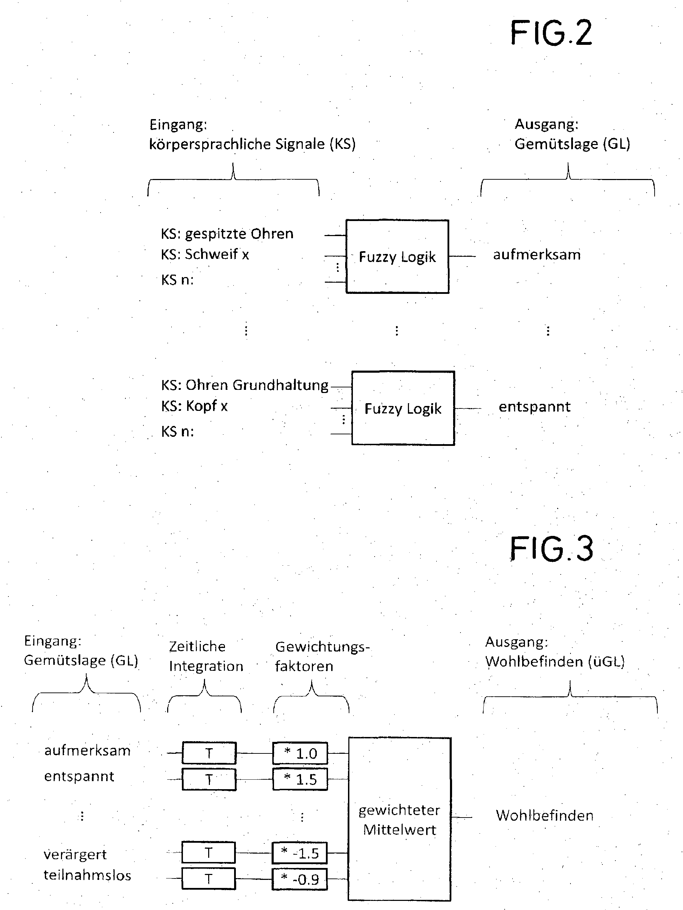

Die Aussage über den Gemütszustand des Pferdes kann die momentane Befindlichkeit des Pferdes widerspiegeln. Es kann jedoch vorgesehen sein, alternativ dazu oder ergänzend dazu eine Aussage über den Gemütszustand während eines bestimmten Zeitintervalls abzugeben, beispielsweise über einen ganzen Tag hinweg oder über eine Woche, einen Monat, ein halbes Jahr o. dgl. Dabei kann diese Aussage entweder zusammenfassend in Form einer so genannten "übergeordneten Gemütslage" bilanzierend ausgegeben werden, also mit einer einzigen Aussage, wie "sehr gut", "gut", "befriedigend", "ausreichend" oder "schlecht", wobei diese zusammenfassende, bilanzierende Aussage die unterschiedlichsten, differenziert erfassten Gemütszustände in einer über dieses bestimmte Zeitintervall integrierten Art enthält und eine Gewichtung dieser unterschiedlichen Gemütszustände berücksichtigt. Darauf wird anhand der Fig. 3 später näher eingegangen.

Alternativ zu dieser zusammengefassten Aussage kann die über ein bestimmtes Zeitintervall getroffene Aussage differenziert ausgegeben werden für die unterschiedlichsten Gemütszustände, so dass beispielsweise angezeigt wird, einen wie großen zeitlichen Raum ein bestimmter Gemütszustand an diesem gewählten Zeitintervall einnahm. Darauf wird anhand der Fig. 2 später näher eingegangen.

Dabei kann vorgesehen sein, diese Aussage über den Gemütszustand bzw. über die mehreren unterschiedlichen Gemütszustände während des Zeitintervalls nicht nur blockartig, als Ergebnis, anzuzeigen, sondern als Verlauf während des Zeitintervalls auszugeben. Auf diese Weise kann beispielsweise erkannt werden, dass regelmäßig zu bestimmten Zeiten die Gemütslage des Pferdes besonders positiv oder besonders negativ ist, beispielsweise im Kontakt mit anderen Pferden, im Kontakt mit einem Pferdepfleger oder Trainer, zu Fütterungszeiten oder bei ähnlichen Umgebungseinflüssen, so dass dementsprechend gezielt Maßnahmen ergriffen werden können, um negative Gemütslagen des Pferdes zu minimieren.

Es ist für viele Personengruppen von Interesse, eine Aussage über die Gemütslage des Pferdes zu erhalten:
Der Pferdebesitzer beispielsweise ist vielfach den größten Teil des Tages weit von seinem Pferd entfernt, und die Kontrolle des Pferdes und seiner Gemütslage ist dementsprechend zeitaufwendig. Mithilfe des vorschlagsgemäßen Verfahrens kann dem Pferdebesitzer auch über große Entfernungen hinweg die Gemütslage seines Tieres angezeigt werden, so dass der Pferdebesitzer beispielsweise sehen kann, wie es seinem Pferd momentan geht.

Wenn das Pferd für sportliche Aktivitäten genutzt werden soll, so ist es bekannt, dass beispielsweise der Stressievel die Leistungsfähigkeit des Pferdes unmittelbar beeinflusst. Dementsprechend kann durch Eliminieren negativer Einflüsse, welche die Gemütslage des Pferdes zum Negativen beeinflussen, die Leistungsfähigkeit des Pferdes verbessert werden, was im Interesse des Pferde-Trainers liegt.

Für den Reitstall, bei dem das Pferd eingestellt ist, bietet das vorschlagsgemäße Verfahren die Möglichkeit, die Qualität der Versorgung des Pferdes zu dokumentieren. Insbesondere bietet sich die Möglichkeit, Änderungen bei der Unterbringung des Pferdes vorzunehmen, beispielsweise das Pferd in einer bestimmten Gruppe mit anderen Pferden auf eine Weide zu lassen oder bestimmte andere als negativ erkannte Einflüsse auf das Pferd zu verringern bzw. positive Einflüsse zu verstärken.

Für den Tierarzt bietet das vorschlagsgemäße Verfahren die Möglichkeit, Krankheiten möglichst früh zu erkennen, z. B. ein Lahmen des Pferdes oder Koliken, oder auch den Heilungsprozess des Pferdes nach einer Erkrankung bzw. während einer Behandlung zu überwachen bzw. zu dokumentieren.

Abgesehen von der Aussagekraft, die der Stellung der Ohren des Pferdes zukommt, kann vorzugsweise auch die Stellung bzw. die Bewegung des Schweifs erfasst werden. Je höher der Schweif getragen wird, umso wachsamer ist das Pferd. Dies kann für Aktivität und Tatendrang im positiven Sinne, ggf. jedoch auch für Aggressivität im negativen Sinne stehen. Je schlaffer der Schweif herunterhängt, desto mehr empfindet das Pferd Müdigkeit, Erschöpfung, Angst oder Schmerz. So können beispielsweise folgende Stellungen bzw. Bewegungen des Schweifs unterschieden werden: Wenn der Schweif gesenkt wird, empfindet das Pferd Unterwürfigkeit, Müdigkeit oder Schwäche. Wird der Schweif hingegen angepresst, empfindet das Pferd Angst. Steht der Schweif stärker, so ist die Gemütslage des Pferdes aggressiv oder angespannt. Bei seitlich schlagenden Schweifbewegungen ist das Pferd verspannt, beispielsweise weil es verstört ist oder Schmerzen empfindet. Heftiges Schlagen des Schweifs lässt erkennen, dass das Pferd wütend ist.

Zusätzlich zu den Sensoren, die der Erfassung der Ohren- bzw. Schweifstellung bzw. der zugehörigen Bewegungen dienen, können weitere Sensoren vorgesehen sein. Die können z. B. die gesamte Körperhaltung des Pferdes oder die Haltung bzw. Bewegung einzelner Gliedmaßen des Pferdes erfassen. Oder sie können dazu dienen, die Ausrichtung des Pferdes in einem vorbestimmten Raum oder im Verhältnis zu anderen Pferden zu erfassen, so dass beispielsweise ermittelt werden kann, wenn ein Pferd eine Sperrhaltung einnimmt, sich also etwa 90°zur Bewegungsrichtung eines anderen Pferdes ausrichtet und dessen Bewegung behindert, denn durch diese Körpersperre zeigt das Pferd seine Dominanz gegenüber dem anderen Pferd, so dass auf diese Weise Unruhe oder Stress bei den beteiligten Pferden aufkommt.

Je nach den verwendeten Sensoren können außerdem Bewegungen der einzelnen Körperteile auch Bewegungen des gesamten Pferdes ermittelt werden, wie beispielsweise der jeweilige Gang des Pferdes, so dass erfasst werden kann, ob das Pferd liegt, steht, schläft, leichte Bewegungen ausführt, frisst oder trinkt, und ob es sich im Schritt, Trab oder Galopp bewegt.

Abweichungen vom normalen Verhalten können erkannt werden, wenn die gesammelten Daten des Pferdes gespeichert und statistisch ausgewertet werden.

Der aktuelle Stresszustand des Pferdes kann anhand der bereits erwähnten Ohrenstellung erfasst werden und ggf. zusätzlich durch eine Herz- oder Atemfrequenzmessung unterstützt werden.

Der Gesundheitszustand des Pferdes kann über die regelmäßig durchgeführten Messungen mit hoher Sicherheit erfasst werden, indem beispielsweise die Symmetrie des Ganges, die Atmung und dergleichen Parameter des Pferdes erfasst werden.

Wenn sich das Pferd auf einer Weide bzw. Koppel befindet und mit anderen Pferden zusammenkommt, so kann beispielsweise aufkommender Stress signalisiert werden, der auf Rangkämpfe innerhalb der Gruppe hinweisen kann, oder auf Koliken o. dgl.

Die erfassten Gemütszustände können zu sehr differenzierten alphanumerischen Auswertungen führen, indem beispielsweise vorbereitete Texte als Analyseergebnis übermittelt werden oder der Gemütszustand als Zahl dargestellt wird, die beispielsweise einem Skalenwert auf einer von "Hervorragend" bis "sehr schlecht" reichenden Gemüts-Skala entspricht.

An Stelle des erwähnten alphanumerisch angezeigten Skalenwerts kann dieser auch analog angezeigt werden, beispielsweise mittels eines Zeigers, der auf einen bestimmten Abschnitt einer Skala zeigt.

Oder die erfassten Gemütszustände können graphisch in Art von Verlaufs-, Block-, Balkendiagrammen o. dgl. angezeigt werden.

Sie können im einfachsten Fall durch einen Farbcode anzeigen, ob es beispielsweise dem Pferd gut geht (durch eine grüne Farbgebung signalisiert) oder schlecht geht (rot) oder ob es dem Pferd, weder besonders gut noch besonders schlecht geht, was durch eine gelbe Farbgebung angezeigt werden kann. Auf diese Weise kann entweder eine für Laien problemlos lesbare, besonders übersichtliche und plakative Aussage über den Gemütszustand des Pferdes getroffen werden, oder es kann insbesondere bei gleichzeitiger Anzeige der Gemütszustände einer Vielzahl von Pferden, beispielsweise im Reitstall oder einer Tierklinik, besonders schnell erfassbar angezeigt werden, wie es den einzelnen Tieren geht und wie die allgemeine Gemütslage im Stall ist, da dies anhand des vorherrschenden Farbeindrucks für sämtliche Tiere gleichzeitig und praktisch "auf einen Blick" erfassbar ist. Die entsprechenden Betreuungspersonen können bei einer derart vereinfachten und schnell erfassbaren Darstellung des Gemütszustandes bei den Pferden, bei denen der Farbcode beispielsweise Rot anzeigt, entweder das Tier aufsuchen oder eine detaillierte Analyse des Pferdes aufrufen, um auf diese Weise detaillierter zu sehen, welche Probleme das Pferd momentan hat.

Das Pferd ist ein Herdentier und kommuniziert in einem großen Maß mithilfe seines Körpers. Die vom Pferd ausgesandten körpersprachlichen Signale können sowohl dynamisch als auch statisch sein, also sowohl durch die Haltung einzelner Körperteile bzw. des gesamten Körpers als auch durch die entsprechenden Bewegungen gegeben werden. Einzelne Körperteile haben dabei eine unterschiedliche Wichtigkeit. Durch entsprechende sensorische Erfassung physiologischer Parameter des Pferdes kann daher eine hochgenaue Aussage über die Gemütslage des Pferdes getroffen werden.

So kann beispielsweise der Körper an sich erfasst werden, also die gesamte Positur des Pferdes. Der Hals kann sensorisch daraufhin analysiert werden, ob er sich in einer angehobenen oder abgesenkten Stellung befindet. Die Ohren werden vorschlagsgemäß sensorisch daraufhin analysiert, ob sie angelegt, aufgestellt, synchron oder verschieden ausgerichtet sind. Der Schweif kann dahingehend analysiert werden, ob er hoch oder hängend ausgerichtet ist. Das Pferd kann daraufhin überprüft werden, ob es mit den Beinen aufstampft. Eine Überwachung der Augen mittels geeigneter Sensoren kann vorgesehen sein, um eine so weit gehende Verdrehung des Auges zu erfassen, dass fast nur noch das Weiße des Auges sichtbar ist. Auch die Bewegungen des Mauls können daraufhin überwacht werden, ob das Maul aufgesperrt wird, was beispielsweise einer Drohhaltung des Pferdes entspricht. Die gesamte Körperspannung, also der Tonus, kann ebenfalls sensorisch erfasst werden, zudem können in an sich bekannter Weise die Atmung und die Atemgeräusche des Pferdes eine Rolle spielen und sensorisch erfasst werden. Insgesamt lassen sich aus den erfassten physiologischen Daten Gemütszustände des Pferdes ableiten, wie z. B. Anspannung, Entspannung, Wut, Unterwürfigkeit, Schläfrigkeit, Misstrauen, Krankheit oder Aufgeregtheit.

Wenn beispielsweise der Kopf kräftig seitlich geschüttelt wird, oder schnelle Aufwärtsbewegungen vollführt, so fühlt sich das Pferd gestört. Wird der Kopf mehrmals hintereinander kurz geduckt, so ist das Pferd neugierig. Geht die Nüsternpartie seitlich hin und her, so will das Pferd aufmuntern. Teilt das Pferd mit seinem Kopf Stupser aus, so will es losgehen oder die Aufmerksamkeit erregen. Bei offenem Mund hingegen ist das Pferd aggressiv. Wenn der Kopf weggedreht wird, so bedeutet dies eine Ablehnung.

Auch die Beine können zur Erfassung der Gemütslage analysiert werden: Werden die Vorderbeine ruckartig nach vorne bewegt, so stellt dies eine Drohhaltung des Pferdes dar. Wird das Hinterbein gehoben, so ist das Pferd bereit, auszuschlagen. Mit einem Bein zu pochen oder zu stampfen bedeutet, dass das Pferd sich belästigt fühlt. Scharrt das Pferd mit den Beinen bzw. Hufen, so lässt das auf eine Frustration des Pferdes schließen.

Durch eine Überwachung des Pferdemauls kann erfasst werden, ob beispielsweise Kaubewegungen erfolgen, die für eine Unterwürfigkeit sprechen, oder ob locker hängende Lippen eine Entspannung des Pferdes signalisieren.

Insbesondere wenn mehrere Pferde mit entsprechenden Sensoren ausgestaltet sind und die Körperhaltung der Pferde nicht nur jeweils für sich, sondern im Verhältnis zueinander erfasst werden kann, lässt sich beispielsweise analysieren, ob das Pferd die weiter oben bereits erwähnte Körpersperre gegenüber einem anderen Pferd aufbaut und Dominanz ausstrahlt, und ob ggf. diese Gemütslage des Pferdes durch Schulterrempler unterstützt und unterstrichen wird, oder ob beispielsweise ein Pferd dem anderen sein Hinterteil zudreht, um zu signalisieren, dass es Pferd in Ruhe gelassen werden will.

Eine besonders einfache Aussage über den Gemütszustand des Pferdes, die beispielsweise - wie weiter oben erwähnt - in einfache grafische, z. B. farbcodierte Signale umgesetzt werden kann, besteht darin, ein Stressniveau des Pferdes in Form eines übergeordneten Gemütszustandes zu bestimmen, was anhand von Fig. 3 später näher erläutert wird. Hierzu werden Anspannung und Entspannung signalisierende Anteile der Körpersprache des Pferdes berücksichtigt, wobei hierzu vorzugsweise die Stellungen bzw. Bewegungen von Ohren und Schweif sowie Hals des Pferdes mit dem Verhalten des Tieres, wie Stehen, Vorwärts- oder Rückwärtsbewegungen, Steigen u. dgl. kombiniert werden können.

Zur Erfassung der sensorisch erfassten Daten kann vorzugsweise eine elektronische Einheit Verwendung finden, welche vorteilhaft mit einem externen Sensor versehen sein kann. Diese Einheit beinhaltet eine Auswerteelektronik für die Sensorsignale, eine eigene Energieversorgung, sowie ein Funkmodul, welches beispielsweise einen verbreiteten Funkstandard wie Bluetooth, Zigbee, GSM o. dgl. benutzt. Der externe Sensor wird mittels beispielsweise vier Drähten an einen Erweiterungsstecker der elektronischen Einheit angeschlossen. Die elektronische Einheit wird im Kopf- oder Stirnbereich des Pferdes platziert und der externe Sensor zur Erfassung der Ohrstellung bzw. Ohrbewegung wird an einem Ohr angebracht, beispielsweise mithilfe der handelsüblichen und ohnehin häufig bei Pferden verwendeten Ohrenschützer, an dem sich der externe Sensor problemlos befestigen lassen kann. Vorzugsweise kann als externer Sensor ein kombinierter Beschleunigungs-/ Magnetosensor Verwendung finden, da derartige Sensoren preisgünstig erhältlich sind, kleine bauliche Abmessungen aufweisen und Messsignale hoher Genauigkeit ermöglichen.

In einer vorteilhaften Ausgestaltung des Verfahrens können auch die Stellungs- bzw. Bewegungsdaten des Schweifs erfasst werden, beispielsweise mithilfe einer zweiten elektronischen Einheit, welches im Bereich des Pferderückens bzw. des Schweifs befestigt wird. Dies ist für das Pferd nicht unangenehm und führt nicht zu untypischen Bewegungen des Schweifs, denn beispielsweise ist es üblich, Lederriemen unter der Schweifwurzel her zu führen, so dass dementsprechend die Sensoranbringung in diesem Bereich für das Pferd ebenso unproblematisch ist wie die Sensoranbringung an den Ohren mittels der ohnehin praxisüblichen Ohrenschützer.

In einer besonders einfachen und preisgünstigen Ausgestaltung des vorschlagsgemäßen Verfahrens kann vorgesehen sein, die Analyse der Körpersprache des Pferdes ausschließlich mithilfe der Sensoren an Ohren und Schweif durchzuführen, so dass beispielsweise auch bei Überwachung einer größeren Anzahl von Pferden das Datenvolumen vorteilhaft gering gehalten werden kann und dementsprechend beispielsweise ein zentraler Server, der sich in dem Verwaltungsgebäude eines Reitstalls befinden kann, die entsprechenden Datenmengen problemlos verarbeiten kann.

Das vorschlagsgemäße Verfahren wird nachfolgend anhand von rein schematischen Zeichnungen näher erläutert. Dabei zeigt bzw. veranschaulicht
- Fig. 1: ein Schema für die Erkennung von körpersprachlichen Signalen anhand von Sensordaten, am Beispiel der Ohren eines Pferdes,
- Fig. 2: die Ermittlung von Gemütslagen anhand von körpersprachlichen Signalen,
- Fig. 3: die Ermittlung von übergeordneten Gemütslagen (üGL) am Beispiel "Wohlbefinden", und
- Fig. 4: zeigt eine Vorrichtung, mit deren Hilfe das vorschlagsgemäße Verfahren durchführbar ist.

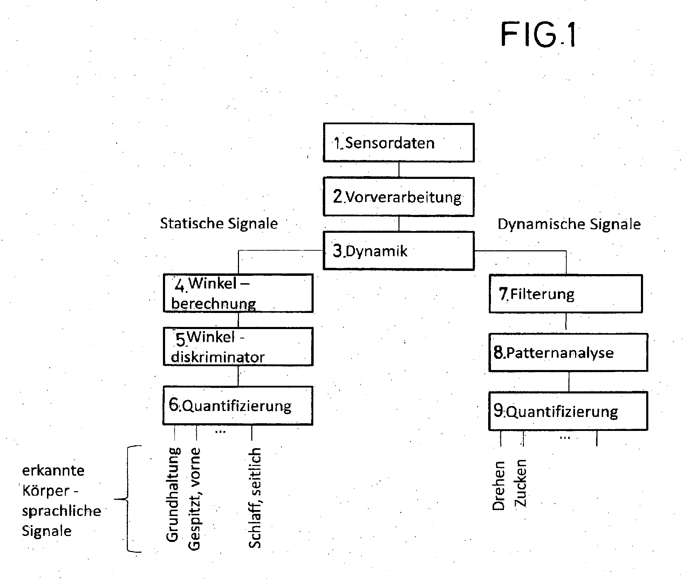

Wie weiter oben bereits erwähnt, erfolgt die Umsetzung von Sensorinformationen in Gemütszustände in mehreren Schritten. Zunächst müssen die verschiedenen körpersprachlichen Signale anhand der Sensordaten erkannt werden. Dann wird jedes erkannte körpersprachliche Signal quantifiziert. Diese Werte werden in einer weiteren Bearbeitungsstufe in detaillierte Gefühle und Stimmungen umgerechnet. Aus diesen einzelnen Gemütslagen werden dann umfassendere Aussagen berechnet, wie z. B. das Wohlbefinden. In Fig. 1 ist das Schema für die Erkennung der körpersprachlichen Signale anhand der Sensordaten am Beispiel Ohren beschrieben. Dabei bedeuten
1. Sensordaten: Um Winkel in allen drei Raumrichtungen (Yaw, Pitch, Roll) bestimmen zu können, ist mindestens eine Kombination aus einem 3-Achsen-Beschleunigungssensor und einem 3-Achsen-Magnetosensor erforderlich. Da in diesem Beispiel die Stellung der Ohren im Vergleich zum Kopf interessieren, ist je eine dieser Kombinationen am Kopf und an den Ohren vorgesehen.
2. Vorverarbeitung: Neben verschiedenen Störunterdrückungsfiltern wird die Differenz zwischen Kopf- und Ohrsensoren gebildet.
3. Dynamik: In der weiteren Analyse wird zwischen statischen und dynamischen Signalen unterschieden. Dazu wird z. B. die Varianz der Datenkanäle berechnet. Je nach Ergebnis erfolgt die Weiterverarbeitung im statischen bzw. dynamischen Zweig der Analyse.
4. Winkelberechnung: Im statischen Teil der Analyse werden aus den Beschleunigungs- und Magnetodaten die Winkel (Yaw, Pitch, Roll) berechnet.
5. Winkeldiskriminator: Für jedes statische körpersprachliche Signal gibt es einen eigenen Diskriminator, in dem der gültige Bereich anhand von Minimal- und Maximalwinkeln für jeden der 3 Raumwinkel beschrieben ist. Befinden sich alle Winkel innerhalb des gültigen Bereiches, wird ein entsprechendes Signal generiert und das körpersprachliche Signal als erkannt angezeigt.
6. Quantifizierung und Normierung: In diesem Schritt wird das erkannte körpersprachliche Signal quantifiziert. Ein Kriterium dafür ist der Abstand der aktuellen Winkel zu den Grenzwinkeln, ein anderer ist die Dauer im Gültigkeitsbereich. Anschließend wird der Wert auf den betrachteten Zeitraum normiert.
7. Filterung für dynamische Signale: Für die nachfolgende Patternanalyse sind zusätzliche Filteroperationen nötig. Dabei kann handelt es sich einerseits um MovingAverage Filter, andererseits um Filter, die die Differenz oder die Summe der Datenkanäle bilden.
8. Patternanalyse: Für jedes dynamische körpersprachliche Signal ist ein eigenes Pattern vorhanden. Bei Signalen mit relativ konstanten Zeitfenstern, wie z. B. dem Zucken der Ohren wird eine Korrelationsanalyse eingesetzt. Als Pattern dient dabei ein typischer Messdatensatz des zu suchenden Ereignisses. Bei weniger genau definierten Bewegungen kommt eine beschreibende Analyse zum Einsatz: beispielsweise wird das Pattern durch eine minimale und maximale zeitliche Abfolge von Extremwerten und den dazwischen liegenden Pegeln in verschiedenen Datenkanälen definiert.
9. Quantifizierung und Normierung der dynamischen Signale: Zur Quantifizierung dient ein oder mehrere Extremwert in einem der Datenkanäle innerhalb des gefunden Patterns. Ein weiteres Kriterium ist die Häufigkeit pro Zeiteinheit. Der Wert wird anschließend auf den betrachteten Zeitbereich normiert

In Fig. 2 wird die Ermittlung von Gemütslagen anhand von körpersprachlichen Signalen erläutert. Dabei werden die körpersprachlichen Signale von verschieden Bereichen wie z. B. Ohren, Schweif, Kopf, Hals zusammengefasst. Dies geschieht beispielhaft mit Fuzzy Logic Modulen. Dabei werden für jede Gemütslage eigene Membership Funktionen und Regelsätze definiert. Ein Regelsatz ist nach dem Schema "Wenn 'Ohren gespitzt' = 'start' und 'Schweif hoch' = 'start', dann ist 'Aufmerksam' = 'noch'. Die Umsetzung der quantifizierten körpersprachlichen Signale in die Bedeutung 'stark' geschieht durch die Membership Funktionen. Der Ausgang dieser Module, die Gemütslage, wird als Wahrscheinlichkeitswert zwischen 0 und 1 dargestellt.

In einem dritten Verarbeitungsschritt werden die verschiedenen Gemütslagen zu einer übergeordneten Gemütslage (üGL) zusammengefasst. Zweck dieser üGL ist es, einen schnellen Überblick über das Befinden des Pferdes zu liefern, z. B. über einen Farbkode. In Fig. 3 ist die Ermittlung von übergeordneten Gemütslagen (üGL) beispielhaft für den Begriff 'Wohlbefinden' dar-gestellt. Die einzelnen Gemütslagen werden dabei über die Zeit integriert und als gewichtete Eingangssignale zu einem Mittelwert zusammengefasst. Die Gewichtung hängt vom üGL-Modul ab und kann sowohl positiv als auch negativ sein. Alternativ kann dieser Verarbeitungsschritt auch durch das in Fig. 2 beschriebenen FuzzyLogic Verfahren berechnet werden.

In dem oben beschriebenen Verfahren, wird die Gemütslage nach allgemeinen Maßstäben, die mehr oder weniger für alle Pferde gelten, berechnet. Manchmal ist es aber wünschenswert, die Analyse mehr an das Individuum anzupassen. Das kann z. B. dadurch geschehen, dass die im Verfahren nach Fig. 1 berechneten quantifizierten körpersprachlichen Signale eines Zeitpunktes zu einem Merkmalsvektor zusammengefasst werden. Parallel dazu wird die interessierende Gemütslage durch einen Experten bewertet und in die Analyse mit einbezogen. Durch eine PCA (principal component analysis) können dann die maßgeblichen körpersprachlichen Signale für die gewählte Gemütslage identifiziert werden. Das weitere Vorgehen entspricht dann der Beschreibung.

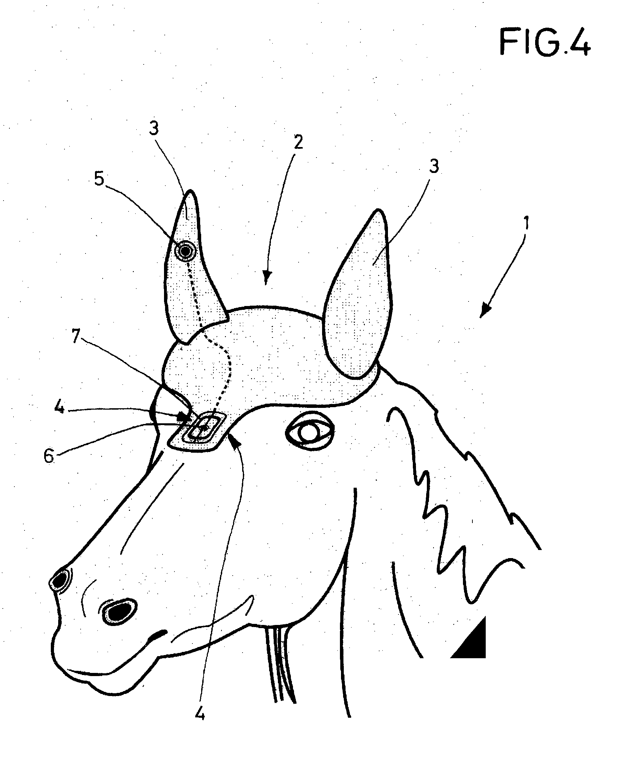

In Fig. 4 ist mit 1 ein lediglich teilweise dargestelltes Pferd bezeichnet, welches einen Ohrschützer 2 trägt, der beide Ohren des Pferdes mittels jeweils einer Ohrhaube 3 bedeckt. Zwischen den Ohren bildet der Ohrschützer 2 einen Stirnlappen 4 aus.

Lediglich rein beispielhaft ist in nur einer der beiden Ohrhauben 3 ein Sensor 5 dargestellt, der die Stellung bzw. Bewegung des betreffenden Ohrs erfasst. Tatsächlich kann in jeder der beiden Ohrhauben 3 jeweils ein solcher Sensor 5 vorgesehen sein.

Am Stirnlappen 4 ist ein weiterer Sensor 6 vorgesehen, mit dessen Hilfe die Stellung bzw. Bewegung des Kopfes erfasst werden kann. Somit kann durch Vergleich der Bewegungen des Kopfes einerseits und der Ohren andererseits präzise errechnet werden, welche Haltung die Ohren am Kopf einnehmen, so dass dementsprechend eine Aussage über den jeweiligen Gemütszustand des Pferdes ermöglicht oder unterstützt wird.

Im Stirnlappen 4 ist weiterhin eine elektronische Einheit 7 vorgesehen, der die Sensordaten der Sensoren 5 und 6 zugeführt werden. Die entsprechende Verbindung zum Sensor 5 ist gestrichelt angedeutet, wobei diese Verbindung drahtgebunden oder drahtlos ausgestaltet sein kann.

Die elektronische Einheit 7 kann zum Zwischenspeichern der Sensordaten dienen, so dass diese Daten später ausgelesen werden können. Die Auswertung der Sensordaten kann dann dementsprechend extern erfolgen. Alternativ kann bereits in der elektronischen Einheit 7 selbst die Auswertung erfolgen, so dass das zu übertragende Datenvolumen erheblich reduziert werden kann.

Die elektronische Einheit 7 kann einen Sender enthalten, welcher die Daten, seien es die Rohdaten oder bereits die Ergebnisse der Auswertung, automatisch zu einer Relaisstation übermittelt. Diese Relaisstation kann im Stall oder an einer Weide bzw. Koppel vorgesehen sein und dazu dienen, die Daten zu einem Empfänger zu übermitteln.

Bei dem Empfänger kann es sich um einen "endgültigen Empfänger" handeln, beispielsweise um einen Trainer, Tierarzt oder Eigentümer des Pferdes, dem die Aussage über den Gemütszustand des Pferdes übermittelt wird. Insbesondere kann diese Aussage mehreren "endgültigen Empfängern" übermittelt werden.

Oder es kann sich bei dem Empfänger um einen zentralen Rechner handeln, der beispielsweise auf dem betreffenden Gestüt bzw. Pferdehof steht, auf dem sich auch das jeweilige Pferd befindet, oder der mit einem als "Expertensystem" bezeichneten Computerprogramm zur Auswertung der Sensordaten versehen ist.

Der erwähnte zentrale Rechner kann dazu dienen, die Sensor-Rohdaten auszuwerten und / oder der mittels einer Anbindung an das Internet, ein Mobilfunknetz oder dergleichen die Rohdaten bzw. die Aussage über den Gemütszustand des Pferdes automatisch zu dem erwähnten "endgültigen Empfänger" weiterzuleiten.

Es kann alternativ auch vorgesehen sein, die Auswertung der übermittelten Daten erst beim endgültigen Empfängervorzunehmen, beispielsweise als Anwendung, die auf einem Smartphone läuft.

## Patentansprüche

1. Verfahren zur Erfassung der Gemütszustände eines Pferdes,
wobei mittels mehrerer Sensoren physiologische Parameter des Pferdes erfasst werden,
und eine Aussage über das Wohlbefinden des Pferdes aus diesen Messwerten abgeleitet wird,
**dadurch gekennzeichnet,**
**dass** die Bewegung und/oder Stellung wenigstens eines Ohrs mittels eines der Sensoren erfasst wird,
wobei anhand der erfassten Sensordaten aus den über die Ohren vermittelten körpersprachlichen Signalen Aussagen über wenigstens zwei qualitativ unterschiedliche Gemütszustände abgeleitet werden,
und **dass** eine Aussage ausgegeben wird, die den Gemütszustand des Pferdes (1) anhand der über die Ohren vermittelten körpersprachlichen Signale wiedergibt.

2. Verfahren nach Anspruch 1,
**dadurch gekennzeichnet,**
**dass** die Bewegung und/oder Stellung des Schweifs sensorisch erfasst wird.

3. Verfahren nach einem der vorhergehenden Ansprüche,
**dadurch gekennzeichnet,**
**dass** der zur Erfassung der Ohren- oder Schweifbewegung und/oder -stellung verwendete Sensor (5) als Bewegungssensor ausgestaltet ist,
und **dass** die Intensität der Bewegung und die Lage im Raum des jeweiligen Körperteils erfasst wird.

4. Verfahren nach einem der vorhergehenden Ansprüche,
**dadurch gekennzeichnet,**
**dass** zur Erfassung der Ohren- oder Schweifbewegung und/oder -stellung ein Magnetfeldsensor verwendet wird.

5. Verfahren nach einem der vorhergehenden Ansprüche,
**dadurch gekennzeichnet,**
**dass** zur Erfassung der Ohren- oder Schweifbewegung und/oder -stellung ein Beschleunigungssensor verwendet wird.

6. Verfahren nach einem der vorhergehenden Ansprüche,
**dadurch gekennzeichnet,**
**dass** ein zusätzlicher Sensor (6) die Bewegungen und / oder die Stellung des Kopfes erfasst, und dass die Ohrbewegung und/oder -stellung relativ zur Kopfbewegung bzw. -stellung ermittelt wird.

7. Verfahren nach einem der vorhergehenden Ansprüche,
**dadurch gekennzeichnet,**
**dass** der zur Erfassung der Ohrenbewegung und/oder -stellung verwendete Sensor (5) in einem Ohrenschützer (2) angeordnet ist und der Ohrenschützer (2) an das Ohr angelegt wird.

8. Verfahren nach Anspruch 7,
**dadurch gekennzeichnet,**
**dass** ein zur Erfassung der Kopfbewegung und/oder-stellung verwendeter zusätzlicher Sensor (6) in dem Teil des Ohrenschützers (2) angeordnet ist, der seine Lage zum Kopf beibehält,
wobei dieser zusätzliche Sensor (6) die Bewegungen und / oder die Stellung des Kopfes erfasst,
und **dass** die Ohrbewegung und/oder -stellung relativ zur Kopfbewegung bzw. -stellung ermittelt wird.

9. Verfahren nach Anspruch 2,
**dadurch gekennzeichnet,**
**dass** der zur Erfassung der Schweifbewegung und/oder -stellung verwendete Sensor (5) an der Schweifwurzel angeordnet wird.

10. Verfahren nach einem der vorhergehenden Ansprüche,
**dadurch gekennzeichnet,**
**dass** die Aussage über den Gemütszustandes des Pferdes (1) differenziert für die unterschiedlichen Gemütszustände ausgegeben wird.

11. Verfahren nach einem der vorhergehenden Ansprüche,
**dadurch gekennzeichnet,**
**dass** eine zusammenfassende Aussage in Form einer übergeordneten Gemütslage ausgegeben wird,
wobei diese zusammenfassende Aussage die unterschiedlichsten, differenziert erfassten Gemütszustände des Pferdes (1) während eines bestimmten Zeitintervalls enthält und eine Gewichtung dieser unterschiedlichen Gemütszustände berücksichtigt.

12. Vorrichtung zur Erfassung der Gemütszustände eines Pferdes (1),
welche einen Ohrclip oder Ohrschützer (2) aufweist,
einen Sensor zur Erfassung der Stellung des Ohres, eine Einheit, die zur Auswertung für die Erkennung und Quantifizierung der über die Ohren vermittelten körpersprachlichen
Signale anhand der erfassten Sensordaten ausgelegt ist,
sowie eine elektronische Einheit (7) zur Speicherung der Sensordaten.

13. Vorrichtung nach Anspruch 12,
**dadurch gekennzeichnet,**
**dass** die Vorrichtung einen zweiten Sensor (6) aufweist, der in der Nachbarschaft vom Ohr am Kopf des Pferdes (1) vorgesehen ist und die Bewegungen und / oder die Stellung des Kopfes erfasst.

## Claims

1. A method for capturing moods of a horse, wherein by means of multiple sensors physiological parameters of the horse are captured and a statement regarding the well-being of the horse is derived from these measurements, **characterized in**
**that** the movement and / or the position of at least one ear is captured by means of at least one of said sensors,
wherein on the basis of the captured sensor data statements are derived from the body language signals mediated by the ears on at least two of qualitatively different moods,
and **that** a statement is provided as output which reflects the mood of the horse (1) on account of the body language signals mediated by the ears.

2. The method of claim 1, **characterized in**
**that** the position of the tail is captured by means of sensors.

3. A method of any of the preceding claims, **characterized in**
**that** the sensor (5) used for capturing the movement and / or the position of the ear or tail is designed as a motion sensor and
**that** the intensity of the movement and the orientation in space of the respective body part is captured.

4. A method of any of the preceding claims, **characterized in**
**that** a magnetic field sensor is used for capturing the movement and / or the position of the ear or tail.

5. A method of any of the preceding claims, **characterized in**
**that** an acceleration sensor is used for capturing the movement and / or the position of the ear or tail.

6. A method of any of the preceding claims, **characterized in**
**that** an additional sensor (6) captures the movement and / or the position of the head
and **that** the movement and / or position of the ear is determined in respect to the movement and / or position of the head.

7. A method of any of the preceding claims, **characterized in**
**that** the sensor for capturing the movement and / or the position of the ear is arranged in an ear protector (2) and that the ear protector (2) is mounted on the ear.

8. The method of claim 7, **characterized in**
**that** an additional sensor (6) to capture the movement and / or the position of the head is arranged in such part of the ear protector (2) which maintains its position in relation to the head,
wherein this additional sensor (6) captures the movement and / or the position of the head,
and **that** the movement and / or position of the ear is determined in respect to the movement and / or position of the head.

9. The method of claim 2, **characterized in**
**that** the sensor (5) for capturing the movement and / or the position of the tail is arranged on the root of the tail.

10. A method of any of the preceding claims, **characterized in**
**that** the statement concerning the mood of the horse (1) is provided as output in a differentiated manner for the different moods.

11. Method of any of the preceding claims, **characterized in**
**that** a summarized statement is provided as output in the form of an overall mood of the horse,
wherein said summarized statement contains the diverse and differentially captured moods of the horse (1) during a specific time interval,
and which takes a weighting of the different moods into account.

12. A device for capturing the moods of a horse (1), comprising:
an ear clip or ear protector (2),
a sensor to capture the position of the ear,
a unit which is configured for the recognition and quantification of the body language signals mediated by the ears on the basis of the captured sensor data,
and an electronic unit (7) to store the sensor data.

13. A device of claim 12, **characterized in**
**that** the device includes a second sensor (6) to be placed in proximity to the ear on the head of the horse (1) and which captures the movements and/or the position of the head.

## Revendications

1. Procédé pour saisir les états d'humeur d'un cheval, sachant qu'au moyen de plusieurs capteurs sont saisis des paramètres physiologiques du cheval et qu'il est dérivé de ces valeurs mesurées un renseignement sur le bien-être du cheval, **caractérisé en ce que** le mouvement et/ou la position d'au moins une oreille est déterminée au moyen de l'un des capteurs, sachant qu'à l'aide des données saisies par capteur, on dérive des signaux du langage corporel communiqués via les oreilles, des renseignements sur au moins deux états d'humeur qualitativement différents, et **en ce qu'**un renseignement est émis qui restitue l'état d'humeur du cheval (1) à l'aide des signaux du langage corporel communiqués via les oreilles.

2. Procédé selon la revendication 1, **caractérisé en ce que** le mouvement et/ou la position de la queue sont saisis par des capteurs.

3. Procédé selon l'une des revendications précédentes, **caractérisé en ce que** le capteur (5) servant à saisir les mouvements et/ou la position des oreilles ou de la queue est configuré en détecteur de mouvement, et **en ce qu'**est saisie l'intensité du mouvement et la position de la partie respective du corps dans l'espace.

4. Procédé selon l'une des revendications précédentes, **caractérisé en ce que** pour saisir le mouvement et/ou la position des oreilles ou de la queue, on utilise un capteur à champ magnétique.

5. Procédé selon l'une des revendications précédentes, **caractérisé en ce que** pour saisir le mouvement et/ou la position des oreilles ou de la queue on utilise un capteur accélérométrique.

6. Procédé selon l'une des revendications précédentes, **caractérisé en ce qu'**un capteur supplémentaire (6) saisit les mouvements et/ou la position de la tête, et **en ce que** le mouvement et/ou la position des oreilles est déterminé(e) relativement au mouvement et/ou à la position de la tête.

7. Procédé selon l'une des revendications précédentes, **caractérisé en ce que** le capteur (5) servant à saisir le mouvement et/ou la position des oreilles est agencé dans un bonnet protège-oreilles (2) et que ledit bonnet protège-oreilles (2) est appliqué contre l'oreille.

8. Procédé selon la revendication 7, **caractérisé en ce qu'**un capteur supplémentaire (6) servant à saisir le mouvement et/ou la position de la tête est disposé dans la partie du bonnet protège-oreilles (2) conservant sa position par rapport à la tête, sachant que ce capteur supplémentaire (6) saisit les mouvements et/ou la position de la tête, et **en ce que** le mouvement et/ou la position des oreilles est déterminé(e) relativement au mouvement et/ou à la position de la tête.

9. Procédé selon la revendication 2, **caractérisé en ce que** le capteur (5) servant à saisir le mouvement et/ou la position de la queue est agencé à la base de la queue.

10. Procédé selon l'une des revendications précédentes, **caractérisé en ce que** le renseignement sur l'état d'humeur du cheval (1) est émis de façon nuancée pour les différents états d'humeur.

11. Procédé selon l'une des revendications précédentes, **caractérisé en ce qu'**un renseignement récapitulatif est émis sous la forme d'un état d'humeur prédominant, sachant que ce renseignement récapitulatif contient les états d'humeur du cheval (1) les plus divers et saisis de manière nuancée, pendant une période précise, et qu'il tient compte d'une pondération de ces différents états d'humeur.

12. Dispositif servant à saisir les états d'humeur d'un cheval (1), présentant un clip d'oreille ou un bonnet protège-oreilles (2), un capteur pour saisir la position de l'oreille, une unité d'analyse pour détecter et quantifier les signaux du langage corporel, communiqués par les oreilles, à l'aide des données saisies par les capteurs, ainsi que présentant une unité (7) électronique servant à enregistrer les données des capteurs.

13. Dispositif selon la revendication 12, **caractérisé en ce que** le dispositif présente un second capteur (6) prévu sur la tête du cheval (1) au voisinage de l'oreille, et qui capte les mouvements et/ou la position de sa tête.
